Europäisches Patentamt

European Patent Office

Office européen des brevets

(19)

(11) Publication number : **0 539 087 A1**

(12) **EUROPEAN PATENT APPLICATION**

(21) Application number : 92309344.7

(22) Date of filing : 14.10.92

(51) Int. Cl.⁵ : **A61K 37/02, A61K 47/36, A61K 47/38**

(30) Priority : 21.10.91 US 779697

(43) Date of publication of application :
28.04.93 Bulletin 93/17

(84) Designated Contracting States :
AT BE CH DE DK ES FR GB GR IE IT LI LU NL PT SE

(71) Applicant : MERCK & CO. INC.
126, East Lincoln Avenue P.O. Box 2000
Rahway New Jersey 07065-0900 (US)

(72) Inventor : Bondi, Joseph V.
3825 Kratz Road
Collegeville, PA 19426 (US)

Inventor : Tasi, Pei-Kuo
707 Hoover Road
Blue Bell, PA 19422 (US)
Inventor : Middaugh, C. Russell
Rt. 1, Box 298E
Quakertown, PA 18951 (US)
Inventor : Volkin, David B.
5 Belmont Square
Doylestown, PA 18901 (US)
Inventor : Matuszewska, Bozena
1604 Claudia Way
North Wales, PA 19454 (US)

(74) Representative : Thompson, John Dr. et al
Merck & Co., Inc. European Patent
Department Terlings Park Eastwick Road
Harlow, Essex CM20 2QR (GB)

(54) **Stabilized topical acidic fibroblast growth factor formulation.**

(57) A sterile aqueous medicinal composition containing acidic fibroblast growth factor combined with an amount of heparin sufficient to stabilize acidic fibroblast growth factor, with said acidic fibroblast growth factor and heparin combined with a sufficient amount of hydroxyethyl cellulose to form a viscous solution which when dried forms a film, with said acidic fibroblast growth factor, heparin and hydroxyethyl cellulose combined with a chelating agent capable of stabilizing the composition at temperatures higher that 4° C.

FIG. 1

EP 0 539 087 A1

Jouve, 18, rue Saint-Denis, 75001 PARIS

BRIEF DESCRIPTION OF THE DRAWINGS

Figure 1. Effect of heparin concentration on the heat-induced aggregation of aFGF at 40° C. Time course of turbidity formation.

Figure 2. Effect of heparin concentration on the heat-induced aggregation of aFGF at 40° C. Effect of heparin on aFGF thermal stability at 40° C.

BACKGROUND OF THE INVENTION

Acidic fibroblast growth factor is a 15.9 KDa protein and is a potent mitogen and chemotatic agent in vitro for many cells of ectodermal and mesodermal embryonic origin. Acidic fibroblast growth factor is a potent mitogen and chemotactic agent for vascular endothelial cells in vitro and induces new blood vessel growth, or angiogenesis, in vivo, Thomas et al. Proc. Natl. Acad. Sci. USA 82: 6409-6413 (1985). Other cells stimulated by aFGF in vitro include: dermal fibroblasts, Shipley et al., J. Cell Physiol. 138: 511-518 (1989); keratinocytes, Miller-Davis et al., Exp. Cell Res. 179: 595-599 (1988). Thus, aFGF is able to drive the proliferation of the major cellular components of skin.

A distinctive feature of aFGF in vitro mitogenic activity is a dependence on heparin for biological activity and structural integrity. The addition of heparin to the culture medium increases the potency of aFGF and reduces the decline in potency following storage at -80° C, Gospodarowicz et. al., Endocrine Reviews 8: 95-113 (1987). Heparin also inhibits proteolytic digestion of aFGF by trypsin, plasmin and other proteases, Rosengart et al., Biochem. Biophys. Res. Comm. 152: 432-440 ( 1988). Heparin, other glycosaminoglycans and sulfated polysaccharides were compared to acertain their ability to potentiate the mitogenic effect of bovine aFGF on adult human endothelial cell cultures, Thomas et al., J. Cell. Physiol. 140: 439-448 (1989). Endothelial cells were stimulated in the presence heparin, heparan-SO4, Dextran-SO4 (low molecular weight) and Dextran-SO4 (high molecular weight) while there was little stimulation with chondroitin-4-SO4, chondroitin-6-SO4, Dermatan-SO4, Hyaluronic acid, dextran (low molecular weight) and dextran (high molecular weight).

Finkenaur, European Patent Application, Publication No. 267,015, disclosed a method for stabilizing an aqueous medicinal composition containing a polypeptide growth factor (including aFGF) as an active ingredient by incorporating into the composition an amount of a water soluble polysaccharide sufficient to stabilize the growth factor against loss of biological avtivity in the presence of water. The aFGF appears to be stabilized against loss of activity by incorporating aFGF in a water soluble polysaccharide, such as a cellulose derivative. The preferred cellulose stabilizers are hydroxyethylcellulose and hydroxypropyl methylcellulose and it is suggested that these compounds will stabilize aFGF. European Patent Application, Publication No. 312,208 discloses gel formulations containing aFGF combined with heparin or cellulose derivatives such as hydroxypropylmethyl cellulose and methyl cellulose but no combination of the three. European Patent Application, Publication No. 406,856 describes a stabilized aFGF composition which contains aFGF combined with a water-insoluble hydroxypropyl cellulose.

OBJECTS OF THE INVENTION

It is, accordingly, an objective of the present invention to provide a stabilized formulation of aFGF for topical wound healing or tissue repair. A further object is to provide an aFGF formulation that is stable at temperatures above 4° C. Another object is to provide an aFGF formulation which is stable upon storage for periods longer than a month. A further object is to provide a combination of excipients that when mixed with aFGF results in a viscous formulation stable at room temperature and exhibits full mitogenic activity in cell culture and demonstrates biological activity in vivo to accelerate wound healing or tissue repair. Another object is to provide a viscous film of aFGF on a non-horizontal surface which will retain bioactivity following drying of the film.

SUMMARY OF THE INVENTION

A sterile aqueous medicinal composition containing acidic fibroblast growth factor combined with an amount of heparin sufficient to stabilize acidic fibroblast growth factor, with said acidic fibroblast growth factor and heparin combined with a sufficient amount of hydroxyethyl cellulose to form a viscous solution which when dried forms a film, with said acidic fibroblast growth factor, heparin and hydroxyethyl cellulose combined with a chelating agent capable of stabilizing the composition at temperatures higher that 4° C.

DETAILED DESCRIPTION OF THE INVENTION

The present invention relates to a stable topical formulation of acidic fibroblast growth factor (aFGF). This unique formulation consists of aFGF combined with heparin or a heparin like substance, a cellulose derivative and a chelating agent with said combination being useful for topical wound healing and tissue repair. The preferred embodyment of this invention results in a viscous formulation stable at room temperature, exhibits full mitogenic activity in cell culture and demonstrates biological activity (or bioactivity) in vivo to accelerate wound healing.

Human acidic fibroblast growth factor exists in various microheterogeneous forms which are isolated from the various tissue sources that contain aFGF. Microheterogeneous forms as used herein refer to a single gene product, that is a protein produced from a single gene unit of DNA, which is structurally modified following translation. These structural modifications, however, do not result in any significant alterations of biological activity of the polypeptide. Biological activity and biologically active are used interchangeably and are herein defined as the ability of native or recombinant aFGF to stimulate DNA synthesis in quiescent BALB/c 3T3 fibroblasts as described below, to stimulate any of the cell types described in the art or to capo out any of the functions described in the art, most specifically topical wound healing or tissue repair. The modifications may take place either in vivo or during the isolation and purification process. In vivo, modification results in, but is not limited to, acetylation at the N-terminus, proteolysis, glycosylation or phosphorylation. Proteolysis may include exoproteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the polypeptide at specific locations within the amino acid sequence. Similar modifications can occur during the purification process which also results in the production of microheterogeneous forms. The most common modification occurring during purification is proteolysis which is generally held to a minimum by the use of protease inhibitors. Under most conditions a mixture of microheterogeneous forms are present following purification of native aFGF. Native aFGF refers to aFGF isolated and purified from tissues or cells that contain aFGF.

Native human aFGF exists in the following microheterogeneous forms. The most preferred microheterogeneous forms of human aFGF include a 154 amino acid form, a 140 amino acid form and a 139 amino acid form. The amino acid sequence for the 139, 140 and 154 amino acid forms of aFGF are described in U.S. Patent No. 4,868,113 and European Patent Application, Publication No. 259,953. The various forms of aFGF can be synthesized by either recombinant biotechnological procedures as described in European Patent Application, Publication No. 259,953 or purified from human tissue as described by Gimenez- Gallego et al., Biochem. Biophys. Res. Commun. 138: 611-617 (1986). These procedures can also be used to produce any microheterogeneous form of aFGF which is active as a wound healing agent. Recombinant derived, 140 amino acid form is the preferred form of aFGF. It is to be understood that aFGF produced by either process will be free of any contaminating microorganisms and toxins and is considered sterile. It is further noted that the stabilizers and excipients which are added to the aFGF will also be free of microorganisms and toxins. This will assure that the final wound healing formulation will not contaminate any of the wounds on which these formulations are used.

It is further intended that a preferred embodyment of the present invention include recombinant aFGF in any of the microheterogeneous forms described above. Recombinant aFGF as used herein refers to aFGF prepared by the techniques of biotechnology in which the specific DNA for the microheterogeneous aFGF is inserted into a vector which is incorporated into a host cell. The host cell under the proper conditions will produce the required aFGF. If the host cell is bacterial in nature the resultant polypeptide will generally have a methionine residue as the first amino acid at the amino terminus of the polypeptide chain. A most perferred embodyment of this invention is aFGF with a methionine residue at the first position. This results in Met-aFGF with the polypeptide containing 141 amino acids.

The concentration of aFGF in the following formulations is usually within the range of from about 0.1 μg/ml to about 1500 μg/ml of aqueous formulation ( this includes either the initial aqueous formulation or a formulation that has been reconstituted after dehydration). The preferred concentration of aFGF for topical formulation is from about 25 μg to about 800 μg/ml. The most preferred concentration of aFGF for topical formulations is from about 50 μg/ml to about 250 μg/ml.

Homogeneously pure aFGF is not chemically and/or conformationally stable or biologically active without being stabilized. Stabilization as used herein refers to the addition of chemicals capable of interacting directly with aFGF to maintain a stable and biologically active molecule and chemicals which can maintain stability without direct interaction with aFGF. The present invention is a formulation in which at least one of both types of stabilizing chemicals is present.

Acidic fibroblast growth factor is first stabilized by the addition of heparin, heparin like substances or sulfated dextrans. The heparin and heparin like substances include, but are not limited to, bovine heparin, porcine heparin and heparan sulfate while the sulfated dextrans include, but are not limited to, low molecular weight dextran sulfate (average molecular weights of about 8,000) and high molecular weight dextran sulfate (average molecular weights of about 500,000). The preferred first stabilizer is heparin with the most preferred being porcine heparin. The concentration of heparin in the following formulation is usually within the range of form about 0.1 µg/ml to about 15 mg/ml of aqueous formulation. The preferred concentration of heparin is about 0.1 to about 10 times (X) the concentration of aFGF on a weight per weight basis, Copeland et al., Archives of Biochem. and Biophys. 289: 53-61 (1991). The most preferred concentration is about 1 to about 5 X the concentration of aFGF on a weight per weight basis.

Topical formulations of aFGF may require relative long dermal contact dosing and thus require a formulation which prevents loss of the drug due to run off. To achieve these ends aFGF combined with heparin are combined with a polymer which forms a stable viscous solution even after a freeze/thaw cycle at -70° C. An acceptable polymer is one which dissolves easily and forms a viscous solution in both water and phosphate buffered saline. The upper concentration limit is about 1.5 %. The solution (without aFGF) must be able to withstand autoclave sterilization without apparent changes in the inherent properties. The final formulation containing aFGF must withstand freeze-thaw cycles without any significant change in viscosity. Indeed, the ideal excipient will be one which results in an elastic moisture-retaining film that remains on the wound for extended periods of time and releases the aFGF into the wound environment.

The viscous excipients or polymers of the present invention are water soluble polymers such as xanthan gum, alignates or cellulose derivatives such as alkyl celluloses, hydroxylalkyl celluloses and alkylhydroxyalkyl celluloses. Examples of viscous excipients include methyl cellulose, hydroxyethyl cellulose, carboxymethyl cellulose, hydroxypropyl methylcellulose with hydroxyethyl cellulose (HEC) being preferred. The concentration of the preferred excipient, HEC, will range form about 0.25 % to about 2 % on a weight/volume basis with a concentration of about 0.75 % to about 1.25 % being the most preferred.

Formulations containing aFGF in combination with heparin and HEC are not completely stable at temperatures above about 4° C for extended periods of time. Indeed, aFGF plus heparin and HEC becomes unstable at room temperature for extended periods of time and loses both in vitro and in vivo activity following extended storage. The addition of HEC causes a destabilization of the aFGF-heparin combination at temperatures above about 4° C which results in the loss of biological activity. The aFGF-heparin-HEC combination must be further stabilized by compounds that which bind trace metal ions.

This is ertremely important because aFGF contains three free cysteine residues within the polypeptide chain. Thiol groups can be oxidized spontaneously by atmospheric oxygen. The process is catalyzed by trace metal ions, especially copper and iron. Free metal ions must be sequestered and unavailable to catalyze oxidation to maintain stabilized aFGF. One way of blocking the metal ions is to add a chelating agent which can bind the free metal ions. Consequently, an integral part of the present invention is the addition of a chelating agent to the combination of aFGF, heparin and HEC to maintain stability of the combination. The chelating agent may include, but is not limited to, ethylenediaminetetraacetic acid (EDTA) or EDTA salts and ethyleneglycol-bis(B-aminoethyl ether) N,N,N', N'-tetraacetic acid (EGTA) and EGTA salts along with related compounds. The salts include, but are not limited to, calciumdisodium, disodium, tetrasodium, trisodium The preferred chelater is EDTA at a concentration ranging from about 0.05 mM to about 10 mM, with the most preferred concentrations being from about 0.075 mM to about 0.5 mM. Weaker chelating agents such as citrate or histidine are not as effective as EDTA or EGTA and the respectivce salts in stabilizing the aFGF-heparin -HEC combination.

In vitro biological activity of the formulation of the invention is determined by a fibroblast mitogenic assay as described by Linemeyer et al. in European Patent Application, Publication No. 259,953. BALB/c 3T3 A31 fibroblasts (American Type Culture Collection) are plated at about $3 \times 10^5$ cells per 0.32 cm$^2$ area per well in culture media containing about 10% heat-inactivated calf serum and incubated in about 7 % $CO_2$ (pH 7.35 +- 0.05). The cells become fully quiescent by replacing the media with serum free media at about 6, about 24 and about 48 hours later. At about 53 hours after plating samples of the various formulations are added and 0.12 µg of dexamethasone are added, at about 65 hours each well is supplemented with about 0.4 µCi of [methyl-$^3$H]-thymidine (20 Ci/mmole, Dupont) and about 0.6 µg of unlabeled thymidine (Sigma), and at 80 hours the cells are processed for determination of radiolabel incorporation into DNA. Each dose-response point is the average of at least quadruplicate determinations. Other cell types such vascular endothelial cells and corneal endothelial cells can be employed to determine in vitro mitogenicity. The procedures are described in detail by Thomas et al., Proc. Natl. Acad. Sci. USA 82: 6409-6431 (1985). It should be noted that other cell types responsive to aFGF can be employed for equivalent mitogenic assays.

In vitro mitogenicity is a direct correlate of cell division which can result in in vivo tissue growth. It is well known in growth factor research that potent in vitro mitogens are also effective as in vivo growth stimulators. Epidermal growth factor (EGF) is a promoter of keratinocyte growth in vitro and also accelerates epidermal regeneration in vivo, Brown et al., J. Exp. Med. 163: 1319-1324 (1986). Insulin-like growth factors also stimulate division and growth of many different cultured cells and also stimulate growth in vivo , Foresch et al., Ann. Rev. Physiol. 47: 443-467 (1985). Acidic fibroblast growth factor stimulates various cell to divide in vitro, such as fibroblasts, vascular and corneal endothelial cells, as described above, chondrocytes, osteoblasts, myeloblasts, smooth muscle, glial cells and neuroblasts, European Patent Application, Publication No. 319,052. Thomas et al., Proc. Natl. Acad. Sci. USA 82: 6409-6413 (1985), has shown a direct correlation between in vitro mitogenic stimulation and an angiogenic response of chicken egg chrioallantoic membrane which is an example of tissue growth.

Stabilization of aFGF can also be determined by methods other than mitogenecity. Since aFGF is unstable at physiological temperatures (undergoing large structural changes including aggregation which results in a loss of mitogenic and wound healing activity), aFGF stability can be monitoied by evaluating the turbidity of the phamaceutical composition by following the kinetics of temperature induced aggregation of unfolded protein. Aggregation is determined by measurement of the degree of light scattering (turbidity) at about 350 nm in a temperature controlled environment in phosphate buffered saline (about 10 mM phosphate, 120 mM NaCl, pH 7.2).

The kinetics of protein aggregation (turbidity) were monitored by the degree of light scattering at 350 nm using a Perkin Elmer Lambda 6 UV-visible spectrophotometer equipped with a six-cuvette holder. Temperature was controlled by circulating a water/ethylene glycol solution through the cell holder. 0.9 ml of a PBS solution (10 mM phosphate, 120 mM NaCl pH 7.2) was placed into a cuvette and incubated at the appropriate temperature within the spectrophotometer. Once equilibrated, 100 μl of a 1 mg/ml aFGF solution containing the appropriate amount of ligand was added to the cuvette and mixed manually by inversion. The change in optical density at 350 nm over time was continuously monitored. A dead time of 30s was present due to the mixing of the six samples in each experiment. Aggregate formation was irreversible.

Size exclusion high performance liquid chromatography (SEC-HPLC) is also used to monitor aFGF stability by determining the precent protein mass. This technique incorporates phosphate-cesium chloride mobile phase with detection at about 215 nm. Test samples are diluted one to ten (1/10) in mobile phase and the aFGF peak areas are compared to a aFGF standard of known concentration.

Wound healing, in vivo biological activity, in mammals is evaluated in a mouse model employing genetically diabetic C57BL/Ks - db$^+$/db$^+$ female mice (Jackson Laboratory). The assay is a slight modification of an assay described by Marzella et al., Wounds: A Compendium of Clinical Research and Practice, 2: 135-147 (1990). The differences include the use of a single 2 cm$^2$ full thickness wound and the wounds are covered with a polyurethane dressing. Stabilized aFGF formulations are applied to wounds on days 0, about 3 and about 7. Matching placebo formulations are used in a second group of animals. Dressings are changed about every three to four days, at which time wound perimeters are traced for assessment of healing. Comparison of healing rate vs. a placebo control is made and evaluated for statistical significance at the 90 % healed stage.

Stability of the aFGF formulation of this invention is determined by real time stabilization studies in which samples of the formulation are stored at specific temperatures for periods of time up to two years. Accelerated stabilization determinations are made by maintaining the formulation of this invention, with or without HEC, at temperatures between about 40° C and about 55° C.

The stabilized formulations of the present invention are useful in promoting the repair or healing of surface soft tissue wounds resulting from for example burns, cuts, lacerations and cutaneous ulcerations such as those seen in diabetics. Tissue repair or wound healing as used herein is defined as the regeneration of tissue following the stimulation of mesodermal, ectodermal or neuroectodermal derived cells by the formulations. These formulations are most useful for topical administration of the tissue repair formulations.

The stabilized formulations are applied to wounds in need of accelerated repair as a viscous solution and can be either covered or left uncovered. After application there is some drying of the formulation, thus allowing the excipient to form a film which releases the stabilized aFGF. Procedures for the application of the formulations of this invention are well known in the art of topical wound healing.

The following examples illustrate the present invention without, however, limiting the same thereto.

EXAMPLE 1

Evaluation Of Stabilizers By A Turbidametric Assay

Human acidic fibroblast growth factor was produced as described by Linemeyer et al., European Patent Application, Publication No. 259,953, Linemeyer et al., European Patent Application, Publication No. 392,605 and purified by the procedure as described by Yamazaki et al., European Patent Application, Publication No. 408,146. To examine the interactions between aFGF and heparin, a spectrophotometric turbidity assay was developed. This technique monitors the structural stability of aFGF by following the kinetics of temperature induced aggregation of unfolded protein by measurement of the degree of light scattering (turbidity) at 350nm.

The kinetics of protein aggregation were monitored by the degree of light scattering at 350 nm using a Perkin Elmer Lambda 6 UV-visible spectrophotometer equipped with a six-cuvette holder. Temperature was controlled by circulating a water/ethylene glycol solution through the cell holder. The buffer, 0.9 ml of a PBS solution (10 mM phosphate, 120 mM NaCl pH 7.2) was placed into a cuvette and incubated at the appropriate temperature within the spectrophotometer. Once equilibrated, 100 μl of a 1 mg/ml aFGF solution containing the appropriate amount of ligand was added to the cuvette and mixed manually by inversion. The change in optical density at 350 nm over time was continuously monitored. A dead time of 30s was present due to the mixing of the six samples in each experiment.

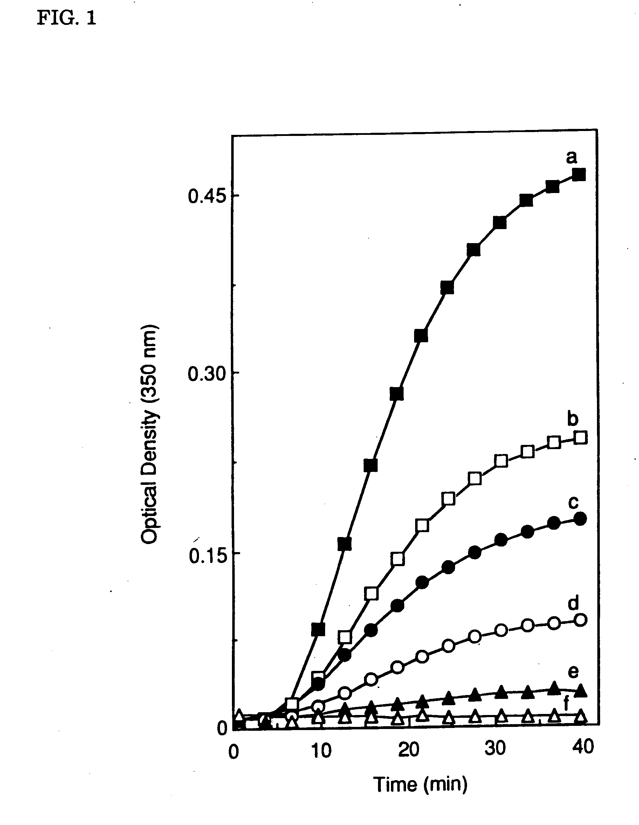

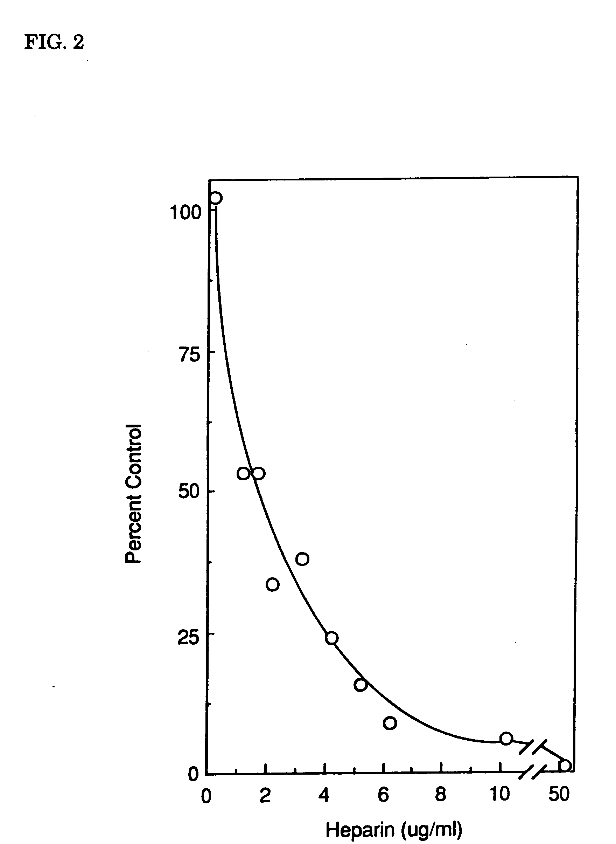

The effect of heparin concentration on the heat-induced aggregation of aFGF at 40° C is illustrated by Figure 1. The time course or rate of aFGF aggregation with and without heparin is shown in Figure 1. Samples contained 100 μg/ml protein in the PBS buffer, pH 7.2 with; (a) no heparin, (b) 1.5 μg/ml heparin, (c) 3 μg/ml heparin, (d) 5 μg/ml heparin, (e) 10 μg/ml heparin and (f) 50 μg/ml heparin. It should be noted that increasing amounts of heparin inhibit the rate of aFGF aggregation. Using the initial linear portion of the slope of turbidity vs. time plots as a measure of the extent of aggregation, the effect of heparin concentration can be quantitatively characterized. Using this approach (as shown in Figure 2), no detectable aggregation of aFGF was observed until the concentration of heparin was approximately 10 μg/ml or less (1:10 weight ratio of heparin to aFGF). The y-axis in Figure 2 represents the maximum rate of turbidity formation ΔOD350 nm/min) normalized to sample (a) of Figure 1.

Similar experiments were carried out to evaluate the stabilizing effect of EDTA in the presence (1/3 X by weight) and absence of heparin at 40° C. The results are shown in the following table.

TABLE 1

| Stabilization Of aFGF With EDTA And Heparin | | | |
|---|---|---|---|
| aFGF μg/ml | Heparin μg/ml | EDTA mM | % Control |
| 100 | 0 | 0 | 100 |
| 100 | 0 | 0.5 | 54 |
| 100 | 0 | 1.0 | 33 |
| 100 | 0 | 2.5 | 13 |
| 100 | 0 | 5.0 | 6 |
| 100 | 0 | 10.0 | 0 |
| 100 | 33 | 0 | 100 |
| 100 | 33 | 0.4 | 45 |
| 100 | 33 | 0.7 | 31 |
| 100 | 33 | 1.0 | 22 |
| 100 | 33 | 2.0 | 33 |
| 100 | 33 | 5.0 | 29 |

The data show that in the absence of heparin, AFGF is increasingly stabilized as the EDTA concentration increases from 0.5 mM to 10 mM. In the presence of heparin, stabilization of aFGF levels off at 0.7- 1.0 mM EDTA.

EXAMPLE 2

Evaluation Of Stability Of aFGF Formulations In Polyethylene Unit Dose Tubes

Acidic fibroblast growth factor was prepared and purified as described in Example 1. Sterile bovine lung heparin (Hepar Industries) was added at the desired concentration of three times by weight of aFGF. This solution is aseptically mixed with a solution of hydroxyethyl cellulose and subdivided into previously sterilized 1 ml polyethylene tubes. The tubes were heat sealed and stored at either 5° or 30° C for various lengths of time. Biological activity of the formulation was determined by a fibroblast mitogenic assay as described by Linemeyer et al. in European Patent Application, Publication No. 259,953. BALB/c 3T3 A31 fibroblasts (American Type Culture Collection) were plated at $3 \times 10^5$ cells per 0.32 cm$^2$ area per well in culture media containing 10% heat-inactivated calf serum and incubated in 7.0 % $CO_2$ (pH 7.35 +- 0.05). The cells become fully quiescent by replacing the media with serum free media at 6, 24 and 48 hours later. At 53 hours after plating samples of the various formulations are added and 0.06 μg of dexamethasone was added, at 65 hours each well was supplemented with 0.4 μCi of [methyl-$^3$H]-thymidine (20 Ci/mmole, Dupont) and 0.6 μg of unlabeled thymidine (Sigma), and at 80 hours the cells were processed for determination of radiolabel incorporation into DNA. Each dose-response point is the average of at least quadruplicate determinations. The result are shown in the following table.

## TABLE 2

### Stabilizing Effect Of Heparin On aFGF

| aFGF Conc. (μg/ml) | Heparin Conc. (μg/ml) | Temp. (° C) | Time Months | % Initial aFGF Conc. | In Vitro Bioactivity (units/mg) |
|---|---|---|---|---|---|
| 50 | 150 | # | 0 | 101 | $3.2 \times 10^6$ |
| | | 5 | 6 | 99 | $2.4 \times 10^6$ |
| | | 5 | 12 | 101 | $3.4 \times 10^6$ |
| | | 5 | 18 | 95 | $4.3 \times 10^6$ |
| | | 5 | 24 | 98 | $2.5 \times 10^6$ |
| | | 30 | 3 | 0 | $0.3 \times 10^{6*}$ |
| 800 | 2400 | # | 0 | 107 | $2.8 \times 10^6$ |
| | | 5 | 6 | 99 | $3.3 \times 10^6$ |
| | | 5 | 12 | 99 | $3.1 \times 10^6$ |
| | | 5 | 18 | 101 | $2.7 \times 10^6$ |
| | | 5 | 24 | 102 | $4.0 \times 10^6$ |
| | | 30 | 2 | 75 | N.D. |
| | | 30 | 6 | 32 | $1.6 \times 10^{6*}$ |

# initial reading

* within acceptable limit of assay

N.D. - no data

Although the formulations show acceptable aFGF stability out to 24 months when stored at 5° C, rapid loss of protein mass and bioactivity is observed during storage at 30° C. This effect is concentration dependent, i.e., the lower the aFGF, the more rapid the loss in protein mass and bioactivity.

EXAMPLE 3

Evaluation Of Stability Of aFGF Formulations Containing EDTA In Polyethylene Unit Dose Tubes

Acidic fibroblast growth factor was prepared and purified as described in Example 1 and formulated as described in Example 2. Disodium EDTA was added to aFGF at a final concentration of either 0.1 mM or 1 mM prior to adding sterile hydroxyethyl cellulose. The formulation was subdivided into previously sterilized 1 ml polyethylene tubes. The tubes were heat sealed and stored at either 5° or 30° C for various lengths of time. Bioactivity was determined by the process described in Example 2. The results are shown in the following table.

TABLE 3

| Stabilizing Effect Of Heparin And EDTA On aFGF | | | | | | |
|---|---|---|---|---|---|---|
| EDTA (mM) | aFGF (µg/ml) | Heparin (µg/ml) | Temp (°C) | Time Months | % Initial Conc. | In Vitro Bioactivity (units/mg) |
| 0.1 | 50 | 150 | 30 | 0 | 105 | $4.3 \times 10^6$ |
| 0.1 | 50 | 150 | 30 | 1 | 99 | $2.0 \times 10^6$ |
| 0.1 | 50 | 150 | 30 | 3 | 104 | $2.7 \times 10^6$ |
| 0.1 | 50 | 150 | 30 | 6 | 100 | $2.9 \times 10^6$ |
| 1.0 | 50 | 150 | 30 | 0 | 107 | $4.0 \times 10^6$ |
| 1.0 | 50 | 150 | 30 | 1 | 97 | $2.4 \times 10^6$ |
| 1.0 | 50 | 150 | 30 | 3 | 93 | $2.8 \times 10^6$ |
| 1.0 | 50 | 150 | 30 | 6 | 97 | $4.9 \times 10^6$ |
| 1.0 | 100 | 300 | 30 | 0 | 100 | $4.3 \times 10^6$ |
| 1.0 | 100 | 300 | 30 | 1 | 115 | $6.0 \times 10^6$ |
| 1.0 | 100 | 300 | 30 | 3 | 104 | $4.9 \times 10^6$ |

In contrast to the data shown in Example 1, both 50 and 100 µg/ml of aFGF show full biological activity and no loss of protein mass when stored for 6 months at 30° C.

EXAMPLE 4

Release Of aFGF From A Viscous 1 % Hydroxyethyl Cellulose Formulation

The formulation of Example 2 was used to evaluate the release of aFGF. Two hundred µl of the HEC formulation containing 0.2 mg aFGF/ml was applied to the donor side of modified Franz diffusion cells (U.S. Patent No. 4,594,884) and the surface on the receptor side was evaluated over time to determine the percent release of aFGF. The amount of acidic fibroblast growth factor was measured by SEC-HPLC. Donor and receptor chambers were separated by a polycarbonate membrane (0.4 µm pore size). The results are listed in the following table.

TABLE 4

| Evaluation Of The Release Of aFGF From A Viscous Solution | |
| --- | --- |
| Time (hr) | % aFGF Released |
| 0 | 0 |
| 1 | 38 |
| 3 | 56 |
| 5 | 72 |
| 7 | 80 |
| 24 | 98 |

The data indicate that aFGF does not bind to the formulation components and that equilibrium with the receptor fluid occurs rapidly.

EXAMPLE 5

Determination Of In Vivo Bioactivity of aFGF Topical Formulations

Acidic fibroblast growth factor as described in Examples 2 and 3 were evaluated to determine the wound healing ability of the stabilized aFGF formulation. Wound healing in mammals was evaluated in a mouse model employing genetically diabetic C57BL/Ks - db$^+$/db$^+$ female mice (Jackson Laboratory). The assay is a slight modification of an assay described by Marzella et al., Wounds: A Compendium of Clinical Research and Practice, 2: 135-147 (1990). The differences include the use of a single 2 cm$^2$ full thickness wound and the wounds were covered with a polyurethane dressing. Stabilized aFGF formulations were applied to wounds on days 0, about 3 and about 7. Matching placebo formulations were used in a second group of animals. Dressings were changed about every three to four days, at which time wound perimeters were traced for assessment of healing. Comparison of healing rate vs. a placebo control was made and evaluated for statistical significance at the 90 % healed stage.

TABLE 5

| aFGF Wound Healing Activity | | |
| --- | --- | --- |
| Formulation | Healing Time 90% (Days) | P |
| Heparin+HEC | 17.38 | |
| aFGF+Heparin+HEC | 13.20 | 0.022 |

The data indicate that aFGF is bioactive in vivo and stimulates the repair or healing of skin wounds.

## Claims

1. A sterile aqueous wound healing composition comprising acidic fibroblast growth factor combined with an amount of a first stabilizer sufficient to stabilize acidic fibroblast growth factor, with said acidic fibroblast growth factor and first stabilizer combined with a sufficient amount of a water soluble excipient selected from the group consisting of cellulose derivatives, xanthan gum and alginate, with said water soluble excipient forming a viscous solution which will permit facile administration to a wound site, with said acidic fibroblast growth factor, said first stabilizer and said soluble excipient combined with a sufficient amount of a second stabilizer capable of stabilizing the composition at temperatures above 4° C.

2. The wound healing composition of claim 1 wherein the acidic fibroblast growth factor is included in a concentration ranging form 10 µg/ml to 1500 µg/ml.

3. The wound healing composition of claim 2 wherein the acidic fibroblast growth factor is included in a concentration ranging form 25 µg/ml to 800 µg/ml.

4. The wound healing composition of claim 1 wherein the first stabilizer is selected form the group consisting of porcine heparin, bovine heparin, heparan sulfate or sulfated dextrans.

5. The wound healing composition of claim 4 wherein the first stabilizer is porcine heparin.

6. The wound healing composition of claim 5 wherein the first stabilizer is included in a concentration ranging from 0.1 µg/ml to 15 mg/ml.

7. The wound healing composition of claim 6 wherein the first stabilizer is included in a concentration of 3 times acidic fibroblast growth factor.

8. The wound healing composition of claim 1 wherein the water soluble cellulose is included in a concentration ranging from 0.25 % to 2 % on a weight/volume basis.

9. The wound healing composition of claim 8 wherein the the water soluble cellulose is included in a concentration ranging form 0.75 % to 1.25 % on a weight/volume basis.

10. The wound healing composition of claim 1 wherein the second stabilizer is a chelating agent.

11. The wound healing composition of claim 10 wherein the chelating agent is selected form the group consisting of ethylenediaminetetraacetic acid and salts and ethyleneglycolbis(B-aminoethyl ether) N,N,N', N'-tetraacetic acid and salts

12. The wound healing composition of claim 11 wherein the ethylenediaminetetraacetic acid is included in a concentration ranging form 0.05 mM to 10 mM.

13. A sterile aqueous wound healing composition comprising acidic fibroblast growth factor combined with an amount of heparin sufficient to stabilize acidic fibroblast growth factor , with said acidic fibroblast growth factor and heparin combined with a sufficient amount of a hydroxyethyl cellulose to allow a viscous solution, with said acidic fibroblast growth factor, heparin and hydroxyethyl cellulose combined with a sufficient amount of the disodium salt of ethylenediaminetetraacetic acid capable of stabilizing the composition at temperatures above 4° C.

14. A sterile aqueous wound healing composition comprising acidic fibroblast growth factor combined with an amount of heparin sufficient to stabilize acidic fibroblast growth factor , with said acidic fibroblast growth factor and heparin combined with a sufficient amount of a hydroxyethyl cellulosesufficient to allow the composition to dry as a film, with said acidic fibroblast growth factor, heparin and hydroxyethyl cellulose combined with a sufficient amount of the disodium salt of ethylenediaminetetraacetic acid capable of stabilizing the composition at temperatures above 4° C.

15. A process for preparing the wound healing composition as claimed in claim 1, which comprises combining a mixture of acidic fibroblast growth factor and said first stabilizer with a solution of said water soluble excipient, and combining the solution thereby obtained with said second stabilizer.

FIG. 1

FIG. 2

European Patent
Office

# EUROPEAN SEARCH REPORT

Application Number

EP 92 30 9344

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,Y | EP-A-0 312 208 (ETHICON)<br>* claims *<br>* page 4, line 40 - line 41 *<br>--- | 1-15 | A61K37/02<br>A61K47/36<br>A61K47/38 |
| D,Y | EP-A-0 406 856 (TAKEDA)<br>* claims *<br>* page 5, line 46 - line 48 *<br>--- | 1-15 | |
| Y | EP-A-0 408 146 (MERCK)<br>* claim 8 *<br>* page 4, line 35 - line 54 *<br>--- | 1-15 | |
| Y | WO-A-9 003 797 (UNIVERSITE PARIS)<br>* claims *<br>* example 1 *<br>--- | 1-15 | |
| Y | EP-A-0 345 660 (TAKEDA)<br>* claims *<br><br>----- | 1-15 | |

TECHNICAL FIELDS
SEARCHED (Int. Cl.5 )

A61K

The present search report has been drawn up for all claims

| Place of search | Date of completion of the search | Examiner |
|---|---|---|
| THE HAGUE | 12 JANUARY 1993 | SCARPONI U. |

EPO FORM 1503 03.82 (P0401)